# EUROPEAN PATENT SPECIFICATION

(11) **EP 3 086 812 B1**
(45) Date of publication and mention of the grant of the patent: **03.07.2019**
(21) Application number: 14756158.3
(22) Date of filing: 07.08.2014
(51) Int. Cl.: A61K 47/18, A61K 47/26, A61K 9/08, C12N 15/113

(54) **NOVEL SUGAR ALCOHOL-BASED COMPOSITIONS FOR DELIVERING NUCLEIC ACID-BASED DRUGS IN VIVO AND IN VITRO**
NEUARTIGE ZUCKERALKOHOLBASIERTE ZUSAMMENSETZUNGEN ZUR FREISETZUNG VON NUKLEINSÄUREBASIERTEN ARZNEIMITTELN IN-VIVO UND IN-VITRO
NOUVELLES COMPOSITIONS À BASE DE POLYOL POUR UNE ADMINISTRATION IN VIVO ET IN VITRO DE MÉDICAMENTS À BASE D'ACIDES NUCLÉIQUES

(30) Priority: 27.12.2013 US 201314142512; 26.01.2014 US 201461931650 P
(43) Date of publication of application: 02.11.2016
(73) Proprietor: Wu, David Ts, Arcadia, California 91006 (US); Lin, Shi-Lung, Arcadia, CA 91007 (US)
(72) Inventor: LIN, Yi-Wen, Cerritos, CA 90703 (US)
(74) Representative: Walker, Ross Thomson
(86) International application number: PCT/US2014/050114
(87) International publication number: WO 2015/099839

(56) References cited:
- US-B1- 6 251 599
- HUI GAO ET AL: "Aminated Linear and Star-Shape Poly(glycerol methacrylate)s: Synthesis and Self-Assembling Properties", BIOMACROMOLECULES, vol. 11, no. 4, 12 April 2010 (2010-04-12) , pages 889-895, XP55150569, ISSN: 1525-7797, DOI: 10.1021/bm901241k

### SEQUENCE LISTING

<110> Lin, Shi-Lung Lin, Yi-Wen
<120> Novel Sugar Alcohol-Based Compositions for Delivering Nucleic Acid-Based Drugs In Vivo and In Vitro
<130> 14P044001EPA00
<150> PCT/US2014/050114
   <151> 2014-08-07
<160> 7
<170> PatentIn version 3.5
<210> 1
   <211> 720
   <212> DNA
   <213> Artificial Sequence
<220>
   <223> chemical synthesis
<400> 1
   aatttttttc ttctaaagtt atgccatttt gttttctttc tcctcagctc taaatactct      60
   gaagtccaaa gaagttgtat gttgggtggg ctcccttcaa ctttaacatg gaagtgcttt     120
   ctgtgacttt aaaagtaagt gcttccatgt tttagtagga gtgaatccaa tttacttctc     180
   caaaatagaa cacgctaacc tcatttgaag ggatcccctt tgctttaaca tgggggtacc     240
   tgctgtgtga aacaaaagta agtgcttcca tgtttcagtg gaggtgtctc caagccagca     300
   caccttttgt tacaaaattt ttttgttatt gtgttttaag gttactaagc ttgttacagg     360
   ttaaaggatt ctaacttttt ccaagactgg gctccccacc acttaaacgt ggatgtactt     420
   gctttgaaac taaagaagta agtgcttcca tgttttggtg atggtaagtc ttctttttac     480
   atttttatta tttttttaga aaataacttt attgtattga ccgcagctca tatatttaag      540
   ctttattttg tatttttaca tctgttaagg ggccccctct actttaacat ggaggcactt     600
   gctgtgacat gacaaaaata agtgcttcca tgtttgagtg tggtggttcc tacctaatca     660
   gcaattgagt taacgcccac actgtgtgca gttcttggct acaggccatt actgttgcta     720
<210> 2
   <211> 69
   <212> RNA
   <213> Artificial Sequence
<220>
   <223> chemical synthesis
<400> 2
   ccaccacuua aacguggaug uacuugcuuu gaaacuaaag aaguaagugc uuccauguuu      60
   uggugaugg                                                              69
<210> 3
   <211> 73
   <212> RNA
   <213> Artificial Sequence
<220>
   <223> chemical synthesis
<400> 3
   gcucccuuca acuuuaacau ggaagugcuu ucugugacuu uaaaaguaag ugcuuccaug      60
   uuuuaguagg agu                                                         73
<210> 4
   <211> 68
   <212> RNA
   <213> Artificial Sequence
<220>
   <223> chemical synthesis
<400> 4
   ccuuugcuuu aacauggggg uaccugcugu gugaaacaaa aguaagugcu uccauguuuc 60
   aguggagg 68
<210> 5
   <211> 68
   <212> RNA
   <213> Artificial Sequence
<220>
   <223> chemical synthesis
<400> 5
   ccucuacuuu aacauggagg cacuugcugu gacaugacaa aaauaagugc uuccauguuu 60
   gagugugg 68
<210> 6
   <211> 23
   <212> RNA
   <213> Artificial Sequence
<220>
   <223> chemical synthesis
<400> 6
   uaagugcuuc cauguuuuag ugu 23
<210> 7
   <211> 23
   <212> RNA
   <213> Artificial Sequence
<220>
   <223> chemical synthesis
<400> 7
   acacuaaaac auggaagcac uua 23

## Description

### FIELD OF INVENTION:

This invention generally relates to a novel chemical composition and its use for formulating RNA- and/or DNA-based drugs/vaccines with modified sugar alcohols and/or sugars into stable complexes for both in-vitro and in-vivo delivery. The invention is defined by the claims. Particularly, the present invention teaches the key ingredients and processes necessary for formulating therapeutic and pharmaceutical nucleic acid compositions, such as microRNA (miRNA) and its precursors/mimics, small hairpin RNAs (shRNA), short interfering RNAs (siRNA), ribozymes, antisense RNAs/DNAs, RNA-DNA hybrids and DNA vectors/vaccines, with novel glycylated sugar alcohols into delivery complexes, which can then be absorbed by cells in vivo and in vitro via an active mechanism, such as endocytosis, for releasing the therapeutic effects of the nucleic acid compositions. The novelty of the present invention is to create positively charged sugar alcohol/sugar-containing compounds for interacting with negatively charged nucleic acid compositions via ionic and/or electrostatic affinity rather than covalent conjugation or hydrogen bonding, so as to preserve the structural integrity of the nucleic acid compositions for better delivery of their drug effects into cells in vivo and in vitro. In addition, the present invention discovered for the first time that chemical compounds like sugar alcohols and sugars can protect small functional nucleic acids, in particular miRNA, shRNA, siRNA and ribozyme molecules, from degradation in vivo as well as in vitro. Therefore, the present invention is not only a composition and its use for delivering nucleic acid-based drugs/vaccines into cells but also a formula for preserving the structural integrity and functional efficacy of these nucleic acid-based drugs and/or vaccines in vivo and in vitro.

### BACKGROUND:

Delivery of functional non-coding RNAs (ncRNA) such as ribozyme, microRNA (miRNA), short hairpin RNA (shRNA) and small interfering RNA (siRNA) is the major obstacle hindering the development of RNA interference (RNAi)-based therapy in vivo. Low penetration and high degradation of these ncRNAs are the problems of delivery. In order to overcome these problems, functional ncRNAs must be protected by a delivery agent that is able to not only preserve their structural integrity but also facilitate their uptake by cells in vivo. Yet, none of previously found liposomal or sugar-based delivery agents can fulfill both needs.

Nucleic acid compositions like ribonucleic acids (RNA) and deoxyribonucleic acids (DNA) are negatively charged molecules and hence tend to attract positively charged materials. On the other hand, the cell membrane consists of phospholipid bilayer, which contains abundant fatty acids and thus is also negatively charged. As a result, naked RNA/DNA will be repelled by the cell membrane and cannot be directly delivered into cells. In order to overcome this problem, one preferred traditional delivery method is liposomal transfection using lipid-based liposomes to encapsulate DNAs/RNAs for intracellular delivery. The mechanism of liposomal transfection is achieved by fusion of liposomes to the phospholipid bilayer of the cell membrane, resulting in passive diffusion of the liposome-encapsulated RNAs/DNAs into the cells. To further improve their delivery efficiency, those liposome molecules are often modified by adding long carbon chains (*i.e.* glycolipids) or positively charged chemical groups, or both, such as polyethylene glycol [PEG; H-(O-CH₂-CH₂)ₙ-OH] (Immordino et al, 2006), glycerol esters (WO2011143237 to Meyering), glycerol monooleate (Pereira et al, 2002; Zhen et al, 2012), and aminated/amino poly(glycerol methacrylate)s (Gao et al, 2010 and 2011). However, due to their limit by passive diffusion, the efficiency of these liposomal methods is generally not comparable to an active delivery method based on endocytosis.

In a liposomal delivery system, glycerol is often used as a polymer linker to connect the long carbon chains of fatty acids and phospholipids, such as monooleate and glycerol esters. Modifications in these long carbon chains can form charged chemical groups to interact with DNA/RNA; yet, those charged carbon chains do not possess any ability (*i.e.* polarity) to protect DNA/RNA from degradation. Alternatively, glycerol also can serve as a side chain in a delivery polymer, such as aminated/amino poly(glycerol methacrylate)s. Those aminated glycerol side chains in such acrylate polymers carry positively charged groups that can form hydrogen bonding (H-bond) with DNA and RNA (Gao et al, 2010). Nevertheless, it is known that the duplex and hairpin structures of DNA and RNA are also formed and maintained by H-bonds. As a result, the H-bonds formed by aminated/amino poly(glycerol methacrylate)s will disturb the structural integrity of DNA/RNA duplexes and hairpins, which are actually required for maintaining the function of many currently known nucleic acid-based drug agents, such as miRNA, shRNA, siRNA, ribozyme and DNA vaccine. Conceivably, none of these liposomal delivery systems can protect DNA and RNA from degradation. Sugar-based delivery is another preferred transfection method, which is designed to improve the low efficiency of liposomal delivery. Sugar-encapsulated DNAs/RNAs can be absorbed by cells via an active endocytosis mechanism, which increases their concentration in cells and hence their functional efficacy as well. A variety of compositions have been used in these sugar-based delivery systems, including sugar-based surfactants (EP0535534 to Nair; WO2009029046 to Kim), poly(sugar acrylate) ploymers (US5,618,933 to Dordick), sugar-grafted liposomes (Banerjee et al, 1996), lipid-protein-sugar particles (WO2002032398 to Kohane et al), poly(glycosylated amino acid)s (Davis et al, 2002), lipoamino acid-/glycopeptide- and/or liposaccharide-conjugants (Blanchfield et al, 2004), pectin/chitosan/lecithin nanoparticles (Morris et al, 2010; Cuna et al, 2006; Graf et al, 2008), sugar-PEG-based polymers (Davis et al, 2010; Bhatia et al, 2011), and boron-saccharide complexes (Ellis et al, 2012). However, none of these sugar-based compositions have been reported to protect the structural integrity of RNA and DNA from degradation. Also, because polysaccharides and sugars do not normally carry any charge, many of these methods still need to be used in conjunction with liposomes in order to encapsulate DNAs/RNAs. As a result, difficult formulation is another problem.

In sum, there is currently no delivery agent that can efficiently deliver RNAs/DNAs into cells while protecting their intact strand structures, particularly duplexes and hairpins, during delivery. Moreover, since these previously developed delivery agents can not been found in any living biological system and have not been tested for in-vivo delivery yet, their safety and in-vivo efficiency are highly uncertain. Therefore, it is highly desirable to have a novel delivery system that not only safely exists in a living system but also is useful for efficiently deliver RNAs/DNAs into cells in vitro as well as in vivo while protecting their intact strand structures, in particular duplexes and hairpins.

### SUMMARY OF THE INVENTION:

Stem cells are like a treasure box containing numerous effective ingredients useful for designing and developing pharmaceutical and therapeutic applications, such as stimulating cell/tissue/organ regeneration, repairing and/or rejuvenating damaged/aged tissues/organs, treating degenerative diseases (*i.e.* diabetes, osteoporosis, Parkinson's and Alzheimer's diseases etc), and preventing tumor/cancer formation, progression and metastasis. Hence, we have used stem cells as a tool for novel drug screening, identification, isolation and production as well as studying the mechanism underlying how stem cells produce and preserve these identified drug ingredients (Chen and Lin, (2013) Recent Patents on Regenerative Medicine 3, 5-16). As a result, the present invention discloses for the first time that chemical compounds containing sugar alcohol- and/or sugar-like structures can protect hairpin-like RNA molecules, in particular microRNA precursors (*i.e.* pri- and/or pre-miRNAs), shRNAs, siRNAs and ribozymes, from degradation in human induced pluripotent stem cells (iPSC) and iPSC-derived embryoid bodies. Due to the structural similarity of all eukaryotic cells, the identified sugar alcohols/sugars may also provide the same protective effect against the degradation of these functional RNA species (*i.e*. microRNAs, shRNAs, siRNAs and ribozymes) in other cell types in vitro and in vivo.

Sugar alcohols are a generic kind of polyol alcohols derived from sugars and also frequently called polyhydric alcohol, polyalcohol, or glycitol. As defined in polymer chemistry, polyols are compounds with multiple active hydroxyl groups available for organic reactions and polymeric polyols are usually in a form of polyethers or polyesters. Most alcohols are white, water-soluble natural occurring materials that are often used in the cosmetic, pharmaceutical and food industries as humectants, thickeners and sweeteners. They are represented by a general chemical formula H(HCHO)ₙ₊₁H, which is different from sugars' H(HCHO)ₙHCO. Also, unlike sugars which tend to form rings, sugar alcohols do not. Yet, they can be dehydrated into cyclic ethers, *e.g*. sorbitol can be dehydrated to isosorbide. The sugar alcohols differ in chain length and have one hydroxyl (OH) group attached to each carbon (C) molecule in the chain. They are further differentiated by their relative orientation (stereochemistry) of these OH groups; for example, mannitol and sorbitol are isomers that share the same chemical formula C₆H₈(OH)₆ but are different in the orientation of the OH group on carbon 2 (C²). The common types of sugar alcohols include, but not limited by, alditol, arabitol, erythritol, fucitol, galactitol, glycerol (or called glycerin), iditol, inositol, isomalt, lactitol, maltitol, mannitol, polyglycitol, sorbitol, threitol, volemitol and xylitol. For the present invention, in addition to sugar alcohols, sugars such as glucose, fructose, galactose, sucrose, and lactose can also be used; alternatively, some sugars can be used to replace sugar alcohols, such as glucose, fructose, galactose, sucrose, and lactose.

Nucleic acid molecules like deoxyribonucleic acids (DNA) and ribonucleic acids (RNA) are negatively charged materials due to the phosphodiester linkage between consecutive nucleotides and thus they tend to interact with positively charged compounds. However, ions in the water often break down this linkage structure of DNA and particularly RNA strands through a force of hydrolysis and hence cause degradation. To prevent RNA/DNA degradation, alcohol materials are known to carry strong polarity that can expel water molecules out of the strands of RNA and DNA, resulting in stabilizing the structural integrity of RNA and DNA strands. In the present invention, we have used modified sugar alcohols and polysaccharides to encapsulate functional RNAs/DNAs for in-vitro and in-vivo delivery; however, sugar alcohols and sugars usually do not carry any charge or sometimes even carry a slightly negative charge under a low pH condition and hence do not interact well with negatively charged nucleic acids. After studying microRNA miR-302 isolated from human induced pluripotent stem cells (iPSCs), we found that some novel modified sugars/sugar alcohols are always purified together with miR-302 and are required for stabilizing and protecting the hairpin structures of miR-302 precursors from degradation. To find this sugar/sugar alcohol identity, our further studies revealed that glycylation is one of the novel sugar/sugar alcohol modifications that promote and enhance the interactions between intracellular sugars/sugar alcohols and RNAs/DNAs, in particular hairpin-like microRNA precursors (*i.e*. pri-/pre-miRNAs). Accordingly, some other similar modifications such as glycylated amino acid-mediated glycylation and glutamylation may provide the same or similar functionality.

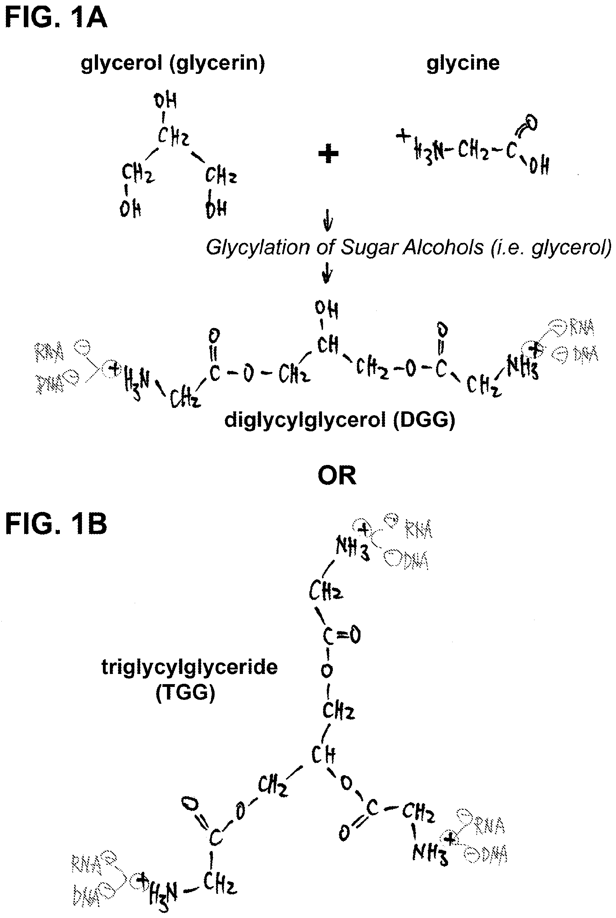

As shown in FIGs. 1A and 1B, a totally novel chemical reaction - glycylation of sugar alcohols - is found to be required for making positively charged sugars/sugar alcohols that form stable delivery complexes with either isolated or recombinant RNAs/DNAs and/or RNA/DNA-like synthetic molecules, which are useful for developing drugs and therapies. Previously, glycylation occurs between amino acids but not in sugars or sugar alcohols. Yet, as defined here, glycylation is a chemical reaction that replaces the hydroxyl (HO-) groups of a sugar alcohol or sugar with glycine's glycyl (NH₂CH₂COO-) groups and thus results in the formation of an ether (R-O-R) linkage between each OH-removed carbon of the sugar/sugar alcohol and the glycyl group. This reaction also involves a process of condensation via dehydration. For example, when glycerol (or called glycerin) is used as a model of sugar alcohol, glycylation of glycerol will generate three kinds of reactive products: one is 1-, or 2-, or 3-monoglycylglycerol (MGG), another is 1,2-, or 2,3-, or 1,3-diglycylglycerol (FIG. 1A; DGG), and the other is 1,2,3-triglycylglyceride (FIG. 1B; TGG). In this model, glycylation can be a partial or completed reaction; for example, MGG and DGG are partially glycylated products, while TGG is a completely glycylated product. All MGG, DGG and TGG are positively charged molecules that interact with negatively charged materials, such as RNA, DNA and any other kind of acidic materials, via ionic/electrostatic affinity and hence useful for intracellular delivery. Due to such charge affinity, the mixture of DNA/RNA and glycylated sugars/sugar alcohols can easily and instantly form encapsulated complexes or polymers. Like natural sugars, these modified (*i.e.* glycylated) sugar alcohols can be absorbed by cells via receptor-mediated endocytosis. Also, sugar alcohols/sugars with higher molecular weights may form higher degree structures of glycylation; yet, the basic function and uptake mechanism of these modified sugar alcohols/sugars are likely similar.

The reaction of glycylation of sugar alcohols/sugars has never been reported before. The present invention herein disclosed for the first time its existence, natural function and the related utilizations thereof. Since glycylated sugar alcohols/sugars can interact with all sorts of small negatively charged materials (including organic or inorganic chemical compounds and drugs) for intracellular delivery, they are useful for developing a variety of therapeutic, pharmaceutical and cosmetic products, devices as well as applications. For instance, they can be used to transduce hydroxylapatite (HA or called hydroxyapatite), filling nanoparticles, hyaluronic acids/kojic acids/ascorbic acids, arbutin, and/or anti-tyrosinase miRNAs/shRNAs/siRNAs/DNAs into epidermal skin cells for cosmetic skin lightening/whitening and/or wrinkle removal. For another example, they can be used to encapsulate and formulate functional microRNAs (*i.e.* pre-miRNA/shRNA) and/or their siRNA mimics as therapeutic drugs/vaccines for treating diseases. To this point, it has been known that microRNAs such as miR-34, miR-146a, miR-142-3p and miR-302 are tumor suppressors which can be used for treating various human tumors/cancers, including but not limited, liver, lung, skin, bone, prostate, breast cancers and brain tumors as well as leukemia [Lin et al., (2008) RNA 14:417-424; (2008) RNA 14:2115-2124; (2010) Cancer Research 70:9473-9482]. Furthermore, these sugar alcohols/sugars may be applied to protect and deliver vector-based DNA/RNA drugs and/or vaccines that are frequently used for gene therapy and anti-viral treatments. As a result, it is conceivable that the present invention can be used in various cosmetic, pharmaceutical and therapeutic designs, devices and applications.

### The Use of Glycylated Sugar Alcohols for Preventing RNA Degradation.

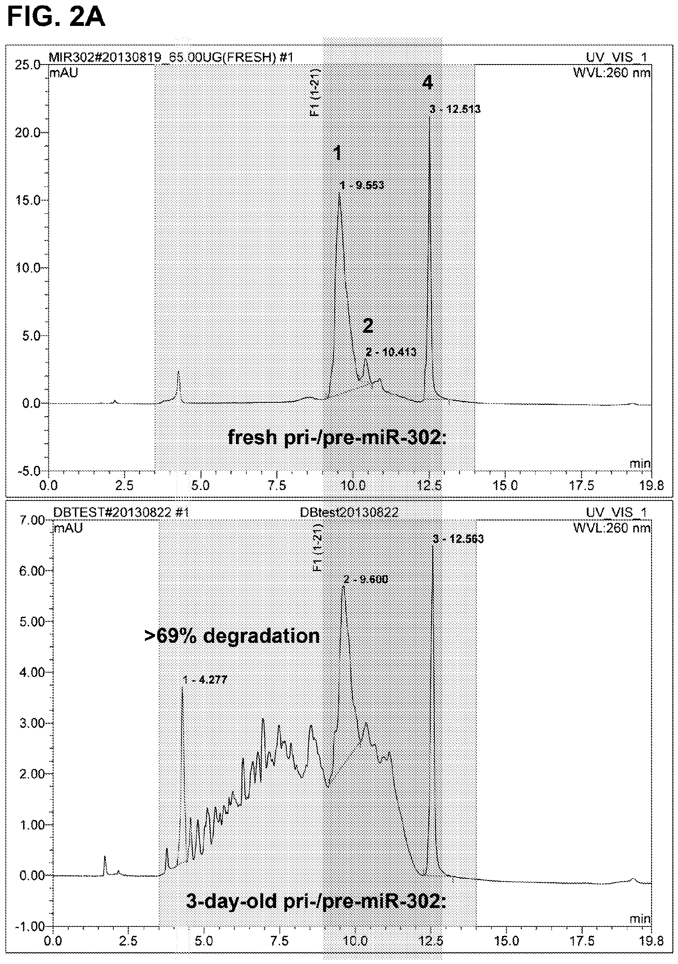

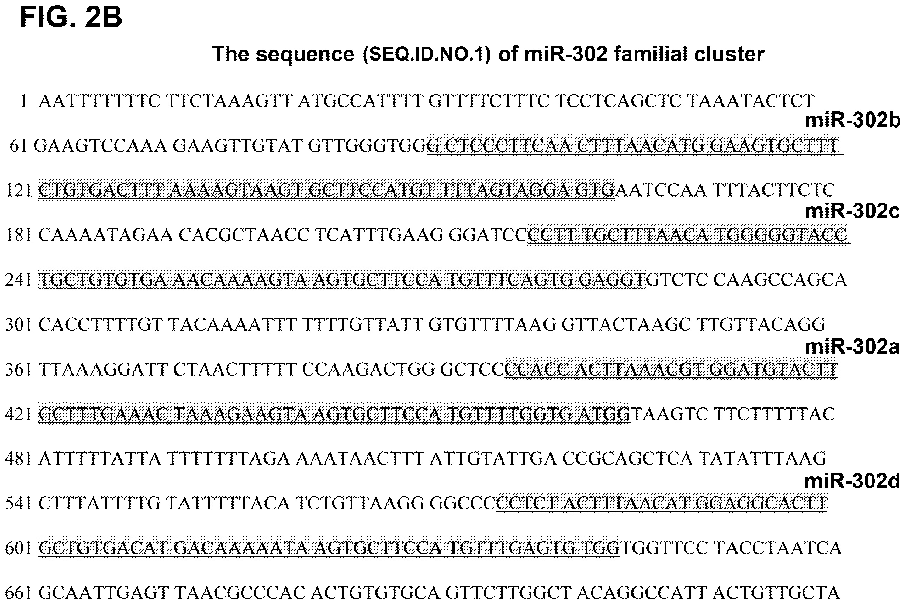

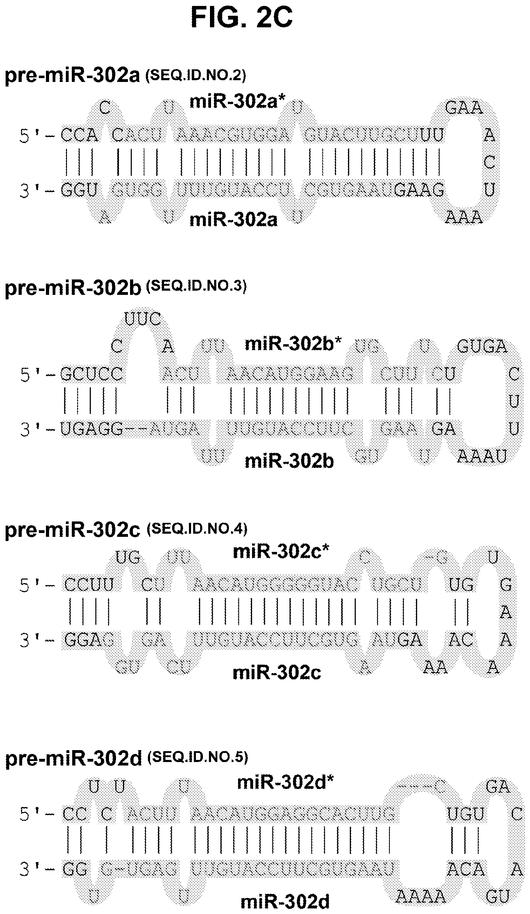

Small non-coding RNAs (ncRNA) are known to be extremely unstable and degradable in vitro and particularly in vivo. The degradation of these ncRNA structures can be measured by high performance liquid chromatography (HPLC) analysis (Example 4) in normal saline (0.9% w/v NaCl), wherein normal saline is used to mimic human body fluids in vivo. For example, as a result shown in FIG. 2A, after comparing fresh and 3-day-old miR-302 precursor samples (Example 1) dissolved in autoclaved normal saline and then store at room temperature, we found that over 69% of pre-miR-302 (mostly 1-hairpin precursors) and pri-miR-302 (4-hairpin cluster) were quickly degraded within three days, which is too short to elicit any RNA interference (RNAi)-associated gene silencing effect. Pri-miR-302, the primary RNA transcript of the miR-302 familial cluster gene (FIG. 2B; SEQ.ID.NO.1), can be processed into one or more pre-miRNA (*i.e.* pre-miR-302) molecules by Drosha/Pasha digestion (to form exonic pre-miRNA), spliceosomal splicing/excision (to form intronic pre-miRNA), or hydrolysis. Pre-miR-302 is the functional form of miR-302 precursor that can be further processed by RNase III Dicer and assembled into an RNA-induced silencing complex (RISC) for eliciting its specific gene silencing effects. As shown in FIG. 2C, we have identified that the processed pre-miR-302 isoforms include pre-miR-302a (SEQ.ID.NO.2), pre-miR-302b (SEQ.ID.NO.3), pre-miR-302c (SEQ.ID.NO.4), and pre-miR-302d (SEQ.ID.NO.5). Yet, due to their fast degradation, these pre-miR-302 molecules can be clearly identified in fresh but barely from 3-day-old samples with microRNA sequencing.

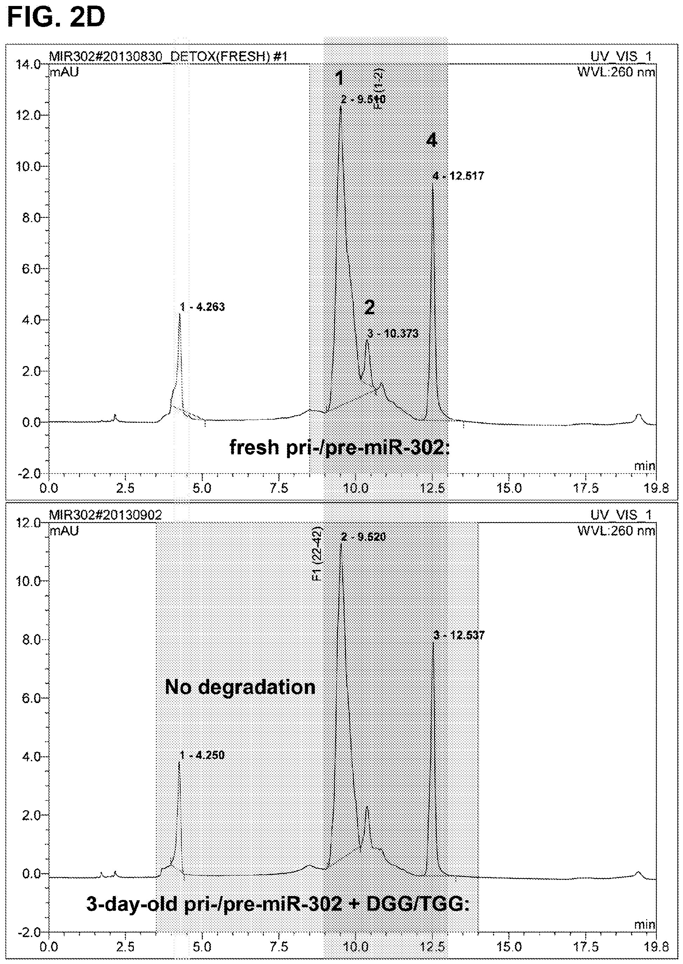

In order to preserve fresh miRNA structures, we added and mixed 0.1 M glycylated sugar alcohols (*i.e.* MGG/DGG/TGG) into fresh miRNA miR-302 samples and then performed the same experimental procedures and HPLC analyses as aforementioned. The final concentration of glycylated sugar alcohol solution used to dissolve RNA/DNA can be ranged from about 0.1 µM to about 10 M, most preferably about 0.05 M to 1.5 M. The maximal solubility of RNA/DNA in MGG/DGG/TGG solution is up to 12-15 mg/mL. As shown in FIG. 2D, we found almost no degradation in these sugar-alcohol-treated samples three days later in normal saline at room temperature. This result has clearly indicated that glycylated sugar alcohols can preserve the integrity of hairpin-like RNA structures and hence prevent the degradation of these hairpin RNAs, such as microRNA precursors (miRNA), small hairpin RNAs (shRNA), short interfering RNAs (siRNA), and ribozymes. Given that RNAi-associated gene silencing effects require at least a three-day period of activation time to be fully effective, the novel sugar alcohol composition found in the present invention is surely useful for stabilizing the structures of these hairpin RNAs long enough to elicit their specific gene silencing effects. Thus, the present invention can be applied to preserve both the structural integrity and functional efficacy of these functional hairpin RNAs in a variety of cosmedical, pharmaceutical and therapeutic products as well as applications.

### Identification of Sugar Alcohol-delivered MicroRNAs in Transfected Cells.

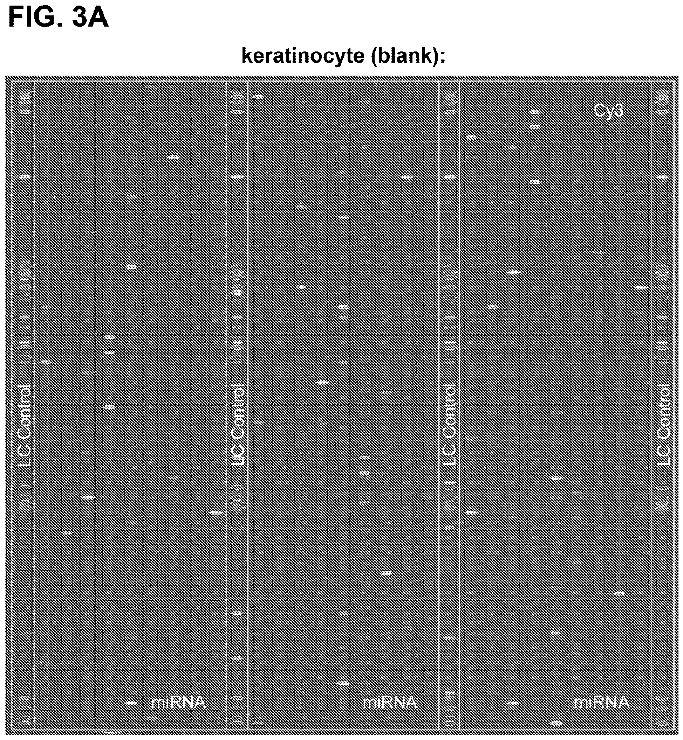

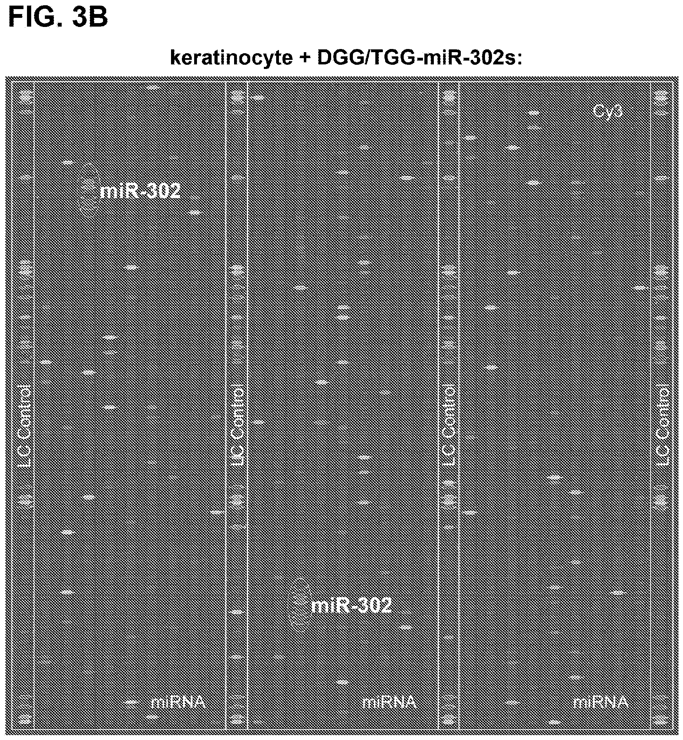

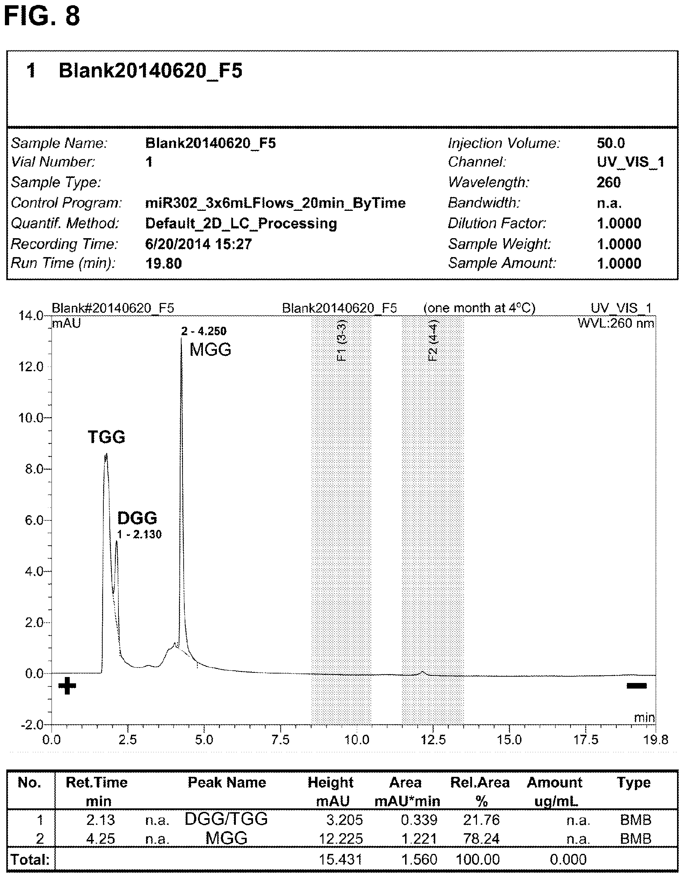

For measuring the deliver efficiency of sugar alcohol-treated nuclei acid compositions into mammalian cells, we isolated and purified miR-302 precursors (*i.e.* pri- and pre-miR-302s) from competent cells transfected with the miR-302 familial cluster gene (Example 1) and then tried to deliver 400 µg of the isolated miR-302 precursors with 1.0 M DGG/TGG into approximately 2∼4 million human keratinocytes grown in 2 ml cell culture medium (Example 3). To increase delivery efficiency, concentrated DGG/TGG was further purified from the mixture solution of MGG/DGG/TGG using HPLC (FIG. 8). As shown in FIGs. 3A and 3B, microarray results (Example 5) revealed that these isolated miR-302 precursors were successfully delivered into the target cells and further processed into mature miR-302 molecules (*e.g*. miR-302a, b, c, d and miR-302a*, b*, c*, d*), indicating that glycylated sugar alcohols not only can efficiently deliver these hairpin RNAs into human cells but also facilitate their assembly into RISCs for eliciting their desired functions. Also, since keratinocytes do not express any miR-302 (FIG. 3A), the highly abundant miR-302 molecules found in the treated keratinocytes (FIG. 3B) must all result from the delivery effect of glycylated sugar alcohols and the delivered miR-302 precursors thereof, suggesting that this highly efficient delivery effect is likely facilitated by an active transporting mechanism such as endocytosis. In nature, such modified sugar alcohols and their derivatives may be absorbed by cells via a receptor-mediated endocytosis mechanism.

### Effects of Sugar Alcohol-delivered Anti-Cancer MicroRNAs against Cancer Cell Growth.

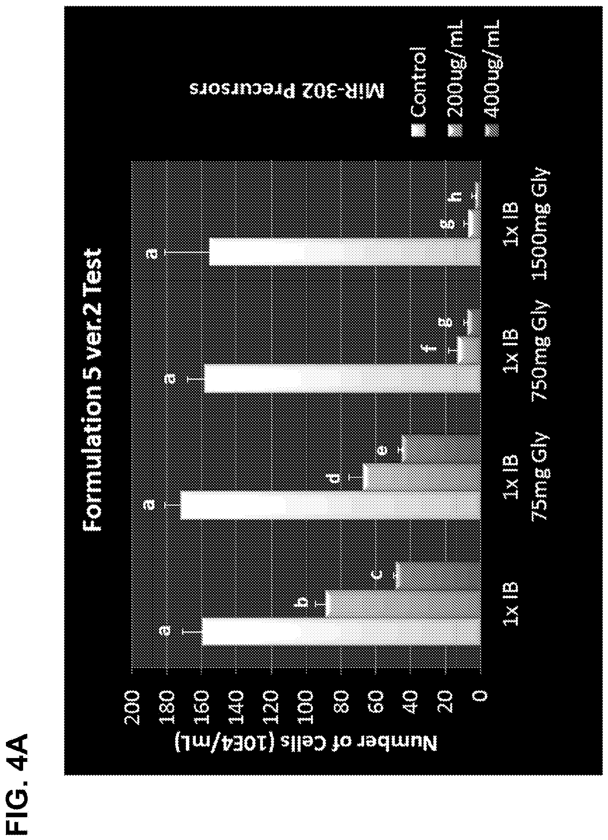

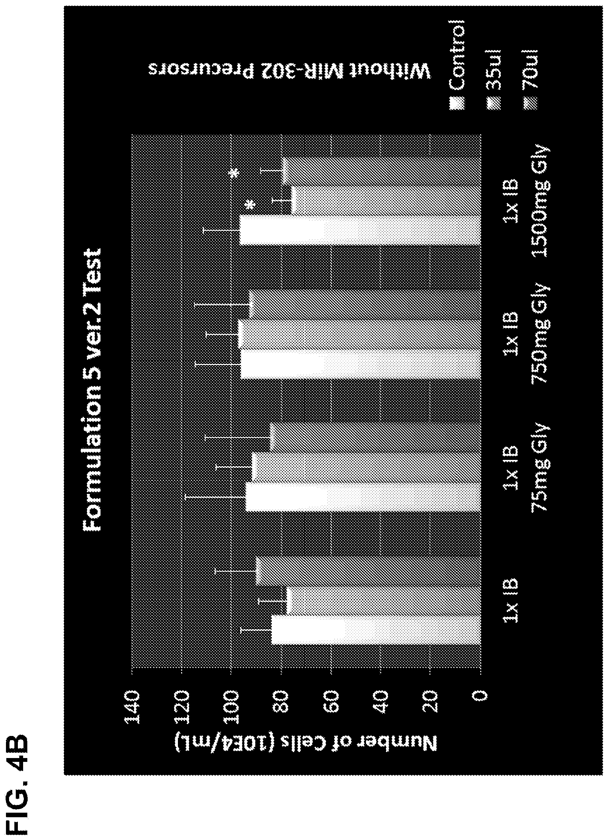

To further observe the sugar alcohol-delivered drug effects, we treated human hepatocellular carcinoma HepG2 cells with MGG/DGG/TGG-encapsulated miR-302 precursors (pri-/pre-miR-302s) using three different final concentrations 0, 200, and 400 µg/ml, respectively. Also, in order to concurrently demonstrate the parallel effects of MGG/DGG/TGG-mediated miR-302 delivery, we encapsulated these pri-/pre-miR-302s with sugar alcohols modified by four different levels of glycylation, including 0, 75, 750, and 1500 mg/10 mL glycine-glycylated glycerol (MGG/DGG/TGG), respectively. Our previous in-vitro studies using vector-based expression have shown that miR-302 can function to inhibit over 95% cancer cell proliferation while only affecting less than 5% to 10% normal cell growth (Lin et al., (2010) Cancer Research 70:9473-9482). As shown in FIG. 4A, the present studies using glycylated sugar alcohol-encapsulated miR-302 delivery showed a similar dose-dependent inhibitory effect on human cancer cell growth, whereas FIG. 4B further shows that the use of glycylated sugar alcohols alone at an equal volume (the equal volume of the same control volume, 35 µl or 70 µl) did not result in any effect or cytotoxicity, indicating that the observed therapy effect of miR-302 is indeed delivered by glycylated sugar alcohols after encapsulating formulation (Example 2).

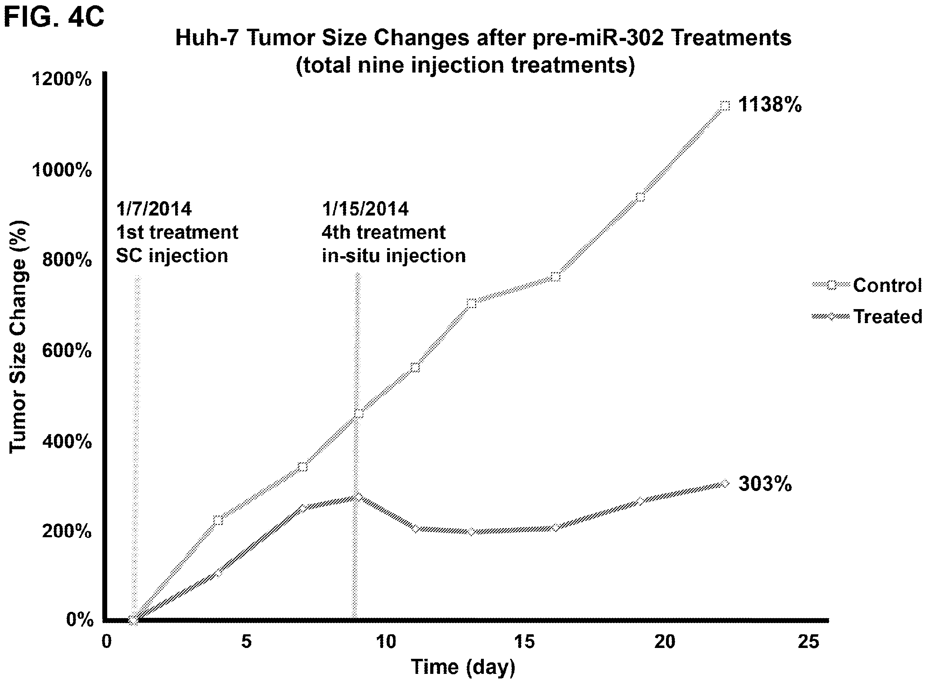

The same MGG/DGG/TGG-delivered tumor suppression effects of miR-302 have been observed in vivo. As shown in FIG. 4C, we injected 250 µg of MGG/DGG/TGG-encapsulated pri-/pre-miR-302s (in 5 µg/µL concentration) into each human Huh-7 liver cancer xenograpft implanted on NOD SCID nude mice (n = 6 for each group). After nine treatments (2∼4 day interval between two treatments), the therapeutic results showed a significant >73% reduction in the sizes of all treated human cancer xenografts compared to those control xenografts without any treatment. Most notably, while the control cancers grew over 11 folds in size during the three-week experiment time, the treated group only expanded 3 folds, indicating a very beneficial tumor suppression effect for treating fast proliferating cancers and preventing cancer relapse after surgical removal. Hence, the results of FIG. 4C clearly demonstrate the high efficiency of glycylated sugar/sugar alcohol-mediated miRNA/shRNA/siRNA delivery in vivo. Since tumor/cancer cells absorb sugar-like materials much more quickly than normal cells, the glycylated sugar/sugar alcohol-encapsulated miRNAs/shRNAs/siRNAs can be easily uptake by tumors/cancers in vivo. It is also noted that these glycylated sugars/sugar alcohols not only deliver the encapsulated RNAs but also successfully introduce the delivered RNA functions into targeted cells.

### In Vivo Distribution of Sugar Alcohol-delivered miRNA/siRNA Mimics in Mouse.

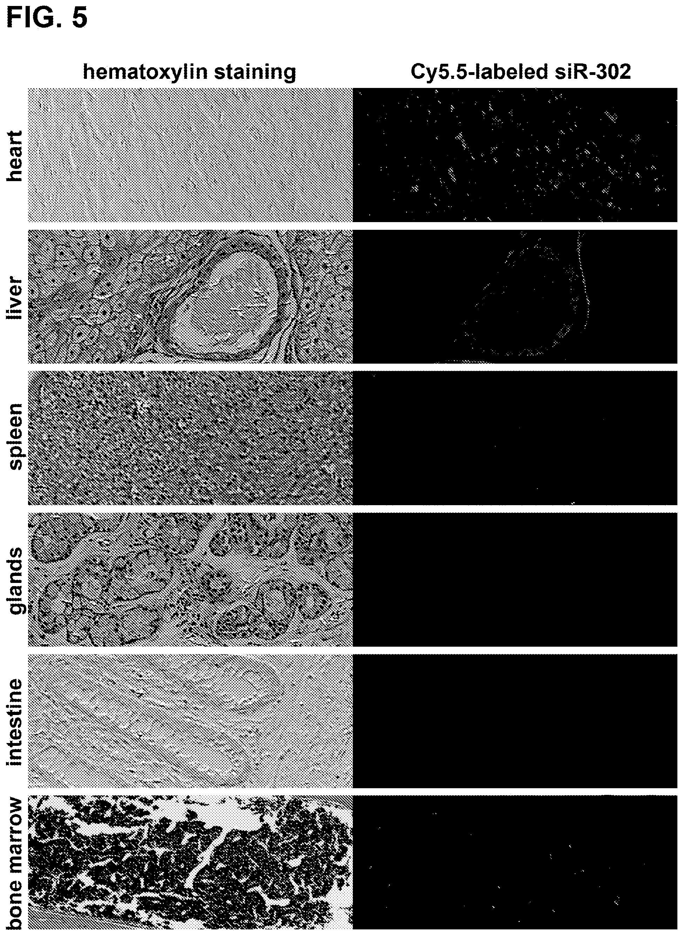

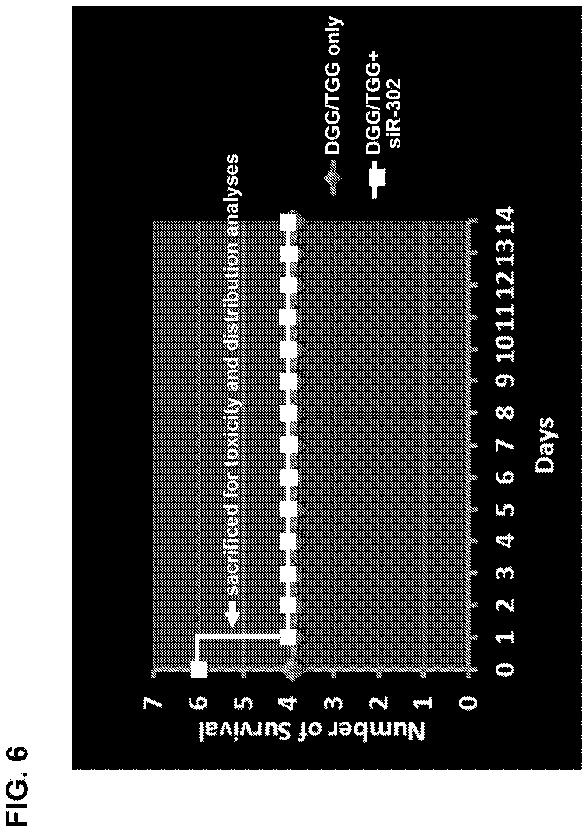

To test the in-vivo deliver efficiency and toxicity of sugar alcohol-encapsulated nucleic acid compositions, we injected 200 µg of synthetic miR-302 mimics (*i.e.* siR-302 from Example 1) dissolved in 200 µl of 1.0 M DGG/TGG into each of C57BL/6J strain mice via tail vein injection (Example 6; n = 6). After 24-hour incubation, we kept 4 mice for further survival rate analysis and sacrificed two for studying the DGG/TGG-delivered siR-302 distribution in vivo. Since the siR-302 molecules were all labeled with infra-red fluorescent dye Cy5.5, we could detect their in-vivo tissue distribution using a bio-imaging system or directly observe their fluorescent signals in mouse tissue sections under a fluorescent microscope. As shown in FIG. 5, after microscopic examination of various tissue/organ sections collected from the injected mice, we found that the majority of Cy5.5-labeled siRNA molecules were delivered into heart, liver, spleen and blood vessel endothelial cells as well as sporadically detected in bone marrow and lung cells, confirming the delivery efficiency of glycylated sugar/sugar alcohol-encapsulated nucleic acid compositions in vivo. On the other hand, the other 4 mice with the same sugar-alcohol-encapsulated siR-302 injection showed no adverse effect during the whole two-week experiment time (FIG. 6), further revealing the in-vivo safety of this novel delivery composition and method.

In sum, we have practically enabled the utilization of a novel sugar alcohol composition not only for preserving the structural integrity of nucleic acid compositions but also for delivering these functional nucleic acid compositions into cells in vitro and in vivo. These functional nucleic acid compositions may include, but not limited, microRNA precursors (miRNA), small hairpin RNAs (shRNA), short interfering RNAs (siRNA), ribozymes, antisense RNAs/DNAs, RNA-DNA hybrids and DNA vectors/vaccines. As safety and efficacy are the two major concerns during drug delivery, the non-toxic and highly tissue-penetrating feature of the presently invented sugar alcohol compositions can overcome these concerns and satisfy the needs of efficient in-vivo delivery for a variety of nucleic acid-based cosmetic, pharmaceutical and therapeutic applications. In particular, it is noted that all materials used to produce these modified (glycylated) sugar alcohol compositions are non-toxic under Food and Drug Administration (FDA) regulations.

### A. Definitions

To facilitate understanding of the invention, a number of terms are defined below:
Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. A nucleoside containing at least one phosphate group bonded to the 3' or 5' position of the pentose is a nucleotide. DNA and RNA are consisted of different types of nucleotide units called deoxyribonucleotide and ribonucleotide, respectively.
Oligonucleotide: a molecule comprised of two or more DNAs and/or RNAs, preferably more than three, and usually more than ten. An oligonucleotide longer than 13 nucleotide monomers is also called polynucleotiude. The exact size will depend on many factors, which in turn depends on the ultimate function or use of the oligonucleotide. The oligonucleotide may be generated in any manner, including chemical synthesis, DNA replication, RNA transcription, reverse transcription, or a combination thereof.
Nucleotide Analog: a purine or pyrimidine nucleotide that differs structurally from adenine (A), thymine (T), guanine (G), cytosine (C), or uracil (U), but is sufficiently similar to substitute for the normal nucleotide in a nucleic acid molecule.
Nucleic Acid Composition: a nucleic acid composition refers to a natural or synthetic oligonucleotide or polynucleotide, such as a DNA or RNA sequence, or a mixed DNA/RNA hybrid sequence, in either a single-stranded or a double-stranded molecular structure.
Gene: a nucleic acid composition whose oligonucleotide or polynucleotide sequence codes for an RNA and/or a polypeptide (protein). A gene can be either RNA or DNA. A gene may encode a non-coding RNA, such as small hairpin RNA (shRNA), microRNA (miRNA), rRNA, tRNA, snoRNA, snRNA, and their RNA precursors as well as derivatives. Alternatively, a gene may encode a protein-coding RNA essential for protein/peptide synthesis, such as messenger RNA (mRNA) and its RNA precursors as well as derivatives. In some cases, a gene may encode a protein-coding RNA that also contains at least a microRNA or shRNA sequence.
Primary RNA Transcript: an RNA sequence that is directly transcribed from a genetic DNA without any RNA processing or modification, which may be selected from the group consisting of hnRNA, pre-mRNA, rRNA, tRNA, snoRNA, snRNA, pri-microRNA (pri-miRNA), viral RNA and their RNA precursors as well as derivatives. After transcription, uracil (U) is substituted for thymine (T).
Precursor messenger RNA (pre-mRNA): primary messenger RNA transcripts of a protein-coding gene, which are produced by eukaryotic type-II RNA polymerase (Pol-II) machineries in eukaryotes through an intracellular mechanism termed transcription. A pre-mRNA sequence contains a 5'-untranslated region (UTR), a 3'-UTR, exons and introns.
Messenger RNA (mRNA): assembly of pre-mRNA exons, which is formed after intron removal by intracellular RNA splicing machineries (spliceosomes) and served as a protein-coding RNA for peptide/protein synthesis. The peptides/proteins encoded by mRNAs include, but not limited, enzymes, growth factors, insulin, antibodies and their analogs/homologs as well as derivatives.
Complementary DNA (cDNA): a single-stranded or double-stranded DNA that contains a sequence complementary to an mRNA sequence and does not contain any intronic sequence.
Sense: a nucleic acid molecule in the same sequence order and composition as the homologous mRNA. The sense conformation is indicated with a "+", "s" or "sense" symbol.
Antisense: a nucleic acid molecule complementary to the respective mRNA molecule. The antisense conformation is indicated as a "-" or "*" symbol or with an "a" or "antisense" in front of the DNA or RNA, *e.g*., "aDNA" or "aRNA".
Base Pair (bp): a partnership of adenine (A) with thymine (T), or of cytosine (C) with guanine (G) in a double stranded DNA molecule. In RNA, uracil (U) is substituted for thymine. Generally the partnership is achieved through hydrogen bonding. For example, a sense nucleotide sequence "5'-A-T-C-G-U-3'"' can form complete base pairing with its antisense sequence "5'-A-C-G-A-T-3'".
5'-end: a terminus lacking a nucleotide at the 5' position of successive nucleotides in which the 5'-hydroxyl group of one nucleotide is joined to the 3'-hydroyl group of the next nucleotide by a phosphodiester linkage. Other groups, such as one or more phosphates, may be present on the terminus.
3'-end: a terminus lacking a nucleotide at the 3' position of successive nucleotides in which the 5'-hydroxyl group of one nucleotide is joined to the 3'-hydroyl group of the next nucleotide by a phosphodiester linkage. Other groups, most often a hydroxyl group, may be present on the terminus.
Template: a nucleic acid molecule being copied by a nucleic acid polymerase. A template can be single-stranded, double-stranded or partially double-stranded, depending on the polymerase. The synthesized copy is complementary to the template, or to at least one strand of a double-stranded or partially double-stranded template. Both RNA and DNA are synthesized in the 5' to 3' direction. The two strands of a nucleic acid duplex are always aligned so that the 5' ends of the two strands are at opposite ends of the duplex (and, by necessity, so then are the 3' ends).
Nucleic Acid Template: a double-stranded DNA molecule, double stranded RNA molecule, hybrid molecules such as DNA-RNA or RNA-DNA hybrid, or single-stranded DNA or RNA molecule.
Conserved: a nucleotide sequence is conserved with respect to a pre-selected (referenced) sequence if it non-randomly hybridizes to an exact complement of the pre-selected sequence.
Homologous or Homology: a term indicating the similarity between a polynucleotide and a gene or mRNA sequence. A nucleic acid sequence may be partially or completely homologous to a particular gene or mRNA sequence, for example. Homology may be expressed as a percentage determined by the number of similar nucleotides over the total number of nucleotides.
Complementary or Complementarity or Complementation: a term used in reference to matched base pairing between two polynucleotides (*i.e.* sequences of an mRNA and a cDNA) related by the aforementioned "base pair (bp)" rules. For example, the sequence "5'-A-G-T-3'" is complementary to the sequence "5'-A-C-T-3"', and also to "5'-A-C-U-3'". Complementation can be between two DNA strands, a DNA and an RNA strand, or between two RNA strands. Complementarity may be "partial (imperfect)" or "complete (perfect)" or "total". Partial complementarity or complementation occurs when only some of the nucleic acid bases are matched according to the base pairing rules. Complete or total complementarity or complementation occurs when the bases are completely or perfectly matched between the nucleic acid strands. The degree of complementarity between nucleic acid strands has significant effects on the efficiency and strength of hybridization between nucleic acid strands. This is of particular importance in amplification reactions, as well as in detection methods that depend on binding between nucleic acids. Percent complementarity or complementation refers to the number of mismatch bases over the total bases in one strand of the nucleic acid. Thus, a 50% complementation means that half of the bases were mismatched and half were matched. Two strands of nucleic acid can be complementary even though the two strands differ in the number of bases. In this situation, the complementation occurs between the portion of the longer strand corresponding to the bases on that strand that pair with the bases on the shorter strand.
Complementary Bases: nucleotides that normally pair up when DNA or RNA adopts a double stranded configuration.
Complementary Nucleotide Sequence: a sequence of nucleotides in a single-stranded molecule of DNA or RNA that is sufficiently complementary to that on another single strand to specifically hybridize between the two strands with consequent hydrogen bonding.
Hybridize and Hybridization: the formation of duplexes between nucleotide sequences which are sufficiently complementary to form complexes via base pairing. Where a primer (or splice template) "hybridizes" with target (template), such complexes (or hybrids) are sufficiently stable to serve the priming function required by a DNA polymerase to initiate DNA synthesis. There is a specific, *i.e.* non-random, interaction between two complementary polynucleotides that can be competitively inhibited.
Posttranscriptional Gene Silencing: a targeted gene knockout or knockdown effect at the level of mRNA degradation or translational suppression, which is usually triggered by either foreign/viral DNA or RNA transgenes or small inhibitory RNAs.
RNA Interference (RNAi): a posttranscriptional gene silencing mechanism in eukaryotes, which can be triggered by small inhibitory RNA molecules such as microRNA (miRNA), small hairpin RNA (shRNA) and small interfering RNA (siRNA). These small RNA molecules usually function as gene silencers, interfering with either expression of intracellular genes or translation of the gene transcripts, or both, that contain certain target sequences either completely or partially complementarity to the small RNAs.
Gene Silencing Effect: a cell response after a gene function is suppressed, consisting of, but not limited, inhibition of oncogene expression, inhibition of cell proliferation, cell cycle arrest, tumor suppression, cancer regression, cancer prevention, cell apoptosis, cell repairing and/or rejuvenation, cell reprogramming, reprogramming diseased cells to a relatively normal state (spontaneous healing), and a combination thereof.
Non-coding RNA: an RNA transcript that cannot be used to synthesize peptides or proteins through intracellular translation machineries. Non-coding RNA includes long and short regulatory RNA molecules such as microRNA (miRNA), small hairpin RNA (shRNA), small interfering RNA (siRNA) and double strand RNA (dsRNA). These regulatory RNA molecules usually function as gene silencers, interfering with expression of intracellular genes containing either completely or partially complementarity to the non-coding RNAs.
MicroRNA (miRNA): single-stranded RNA capable of binding to targeted gene transcripts (mRNAs) that have partial complementarity to the sequence of microRNA. Mature microRNA is usually sized about 17-27 oligonucleotides in length and is able to either directly degrade its intracellular mRNA target(s) or suppress the protein translation of its targeted mRNA(s), depending on the complementarity between the microRNA and its target mRNA(s). Native microRNAs are found in almost all eukaryotes, functioning as a defense against viral infections and allowing regulation of specific gene expression during development of plants and animals. In principle, one microRNA often target multiple target mRNAs to fulfill its full functionality while on the other hand multiple miRNAs may target the same gene transcripts to enhance the effect of gene silencing.
MicroRNA Precursor (pri-/pre-miRNA): hairpin-like single-stranded RNA containing stem-arm and stem-loop regions for interacting with RNase III Dicer endoribonucleases to produce one or multiple mature microRNAs (miRNAs) capable of silencing a targeted gene or a specific group of targeted genes that contain full or partial complementarity to the mature microRNA sequence(s). The stem-arm of a pri-/pre-miRNA can form either a perfectly (100%) or a partially (mis-matched) hybrid duplexes, while the stem-loop connects one end of the stem-arm duplex to form a circle or hairpin-loop conformation required for being assembled into an RNA-induced silencing complex (RISC) with some argonaute proteins (AGO).
Small interfering RNA (siRNA): short double-stranded RNA sized about 18-27 perfectly base-paired ribonucleotide duplexes and capable of degrading target gene transcripts with almost perfect complementarity.
Small or short hairpin RNA (shRNA): single-stranded RNA that contains a pair of partially or completely matched stem-arm nucleotide sequences divided by an unmatched loop oligonucleotide to form a hairpin-like structure. Many natural miRNAs are derived from hairpin-like RNA precursors, namely precursor microRNA (pre-miRNA).
Vector: a recombinant nucleic acid composition such as recombinant DNA (rDNA) capable of movement and residence in different genetic environments. Generally, another nucleic acid is operatively linked therein. The vector can be capable of autonomous replication in a cell in which case the vector and the attached segment is replicated. One type of preferred vector is an episome, *i.e*., a nucleic acid molecule capable of extrachromosomal replication. Preferred vectors are those capable of autonomous replication and expression of nucleic acids. Vectors capable of directing the expression of genes encoding for one or more polypeptides and/or non-coding RNAs are referred to herein as "expression vectors" or "expression-competent vectors". Particularly important vectors allow cloning of cDNA from mRNAs produced using a reverse transcriptase. A vector may contain components consisting of a viral or a type-II RNA polymerase (Pol-II or pol-2) promoter, or both, a Kozak consensus translation initiation site, polyadenylation signals, a plurality of restriction/cloning sites, a pUC origin of replication, a SV40 early promoter for expressing at least an antibiotic resistance gene in replication-competent prokaryotic cells, an optional SV40 origin for replication in mammalian cells, and/or a tetracycline responsive element. The structure of a vector can be a linear or circular form of single- or double-stranded DNA selected form the group consisting of plasmid, viral vector, transposon, retrotransposon, DNA transgene, jumping gene, and a combination thereof.
Promoter: a nucleic acid to which a polymerase molecule recognizes, or perhaps binds to, and initiates RNA transcription. For the purposes of the instant invention, a promoter can be a known polymerase or its cofactor binding site, an enhancer and the like, any sequence that can initiate synthesis of RNA transcripts by a desired polymerase.
RNA Processing: a cellular mechanism responsible for RNA maturation, modification and degradation, including RNA splicing, intron excision, exosome digestion, nonsense-mediated decay (NMD), RNA editing, RNA processing, and a combination thereof.
Targeted Cell: a single or a plurality of human cells selected from the group consisting of a somatic cell, a tissue, a stem cell, a germ-line cell, a teratoma cell, a tumor cell, a cancer cell, and a combination thereof.
Cancerous Tissue: a neoplastic tissue derived from the group consisting of skin cancer, prostate cancer, breast cancer, liver cancer, lung cancer, brain tumor/cancer, lymphoma, leukemia and a combination thereof.
Gene Delivery: a genetic engineering method selected from the group consisting of polysomal transfection, liposomal transfection, chemical transfection, electroporation, viral infection, DNA recombination, transposon insertion, jumping gene insertion, microinjection, gene-gun penetration, and a combination thereof.
Genetic Engineering: a DNA recombination method selected from the group consisting of DNA restriction and ligation, homologous recombination, transgene incorporation, transposon insertion, jumping gene integration, retroviral infection, and a combination thereof.
Tumor Suppression Effect: a cellular anti-tumor and/or anti-cancer mechanism and response consisting of, but not limited, cell cycle attenuation, cell cycle arrest, inhibition of tumor cell growth, inhibition of cell tumorigenecity, inhibition of tumor/cancer cell transformation, induction of tumor/cancer cell apoptosis, induction of normal cell recovery, reprogramming high-grade malignant cancer cells to a more benign low-grade state (tumor regression), and a combination thereof.
Cancer Therapy Effect: a cell response and/or cellular mechanism resulted from a drug treatment, including, but not limited, inhibition of oncogene expression, inhibition of cancer cell proliferation, inhibition of cancer cell invasion and/or migration, inhibition of cancer metastasis, induction of cancer cell death, prevention of tumor/cancer formation, prevention of cancer relapse, suppression of cancer progression, repairing damaged tissue cells, reprogramming high-grade malignant cancers to a more benign low-grade state (cancer regression/remission), and a combination thereof.
Cancer Reversion: a reprogramming mechanism that resets the malignant properties of high-grade cancers back to a relatively normal-like low-grade state in vitro, ex vivo or in vivo.
Glycylation: a chemical reaction that replaces the hydroxyl (HO-) groups of a sugar alcohol or sugar with glycine's or glycylated amino acid's glycyl (NH₂CH₂COO-) groups and thus results in the formation of an ether (R-O-R) linkage between each OH-removed carbon of the sugar alcohol/sugar and the glycyl group of the glycine and/or glycylated amino acid.
Pharmaceutical and Therapeutic Application: a biomedical utilization, treatment method, device and/or apparatus useful for diagnosis, stem cell generation, stem cell research and/or therapy development, tissue/organ repair and/or rejuvenation, wound healing treatment, tumor suppression, cancer therapy and/or prevention, disease treatment, drug production, and a combination thereof.
Prokaryotes: a one-cell organism that lacks a distinct membrane-bound nucleus and has its genetic materials in the form of a continuous strand of DNA.

### B. Compositions and Applications

A composition and its use for formulating nucleic acid compositions with sugar alcohols into delivery complexes for both in-vitro and in-vivo delivery into mammalian cells, comprising: (a) at least a nucleic acid composition with at least a negative charge, and (b) at least a sugar alcohol or sugar modified by glycylation; wherein (a) and (b) are mixed together under a condition to form delivery complexes. The nucleic acid composition can be microRNA precursors (miRNA), small hairpin RNAs (shRNA), short interfering RNAs (siRNA), ribozymes, antisense RNAs/DNAs, RNA-DNA hybrids, DNA vectors/vaccines, and a combination thereof. The condition required for the delivery complex formation is below pH8.0, of which the pH value is preferred to be about pH2.5 to about pH7.0.

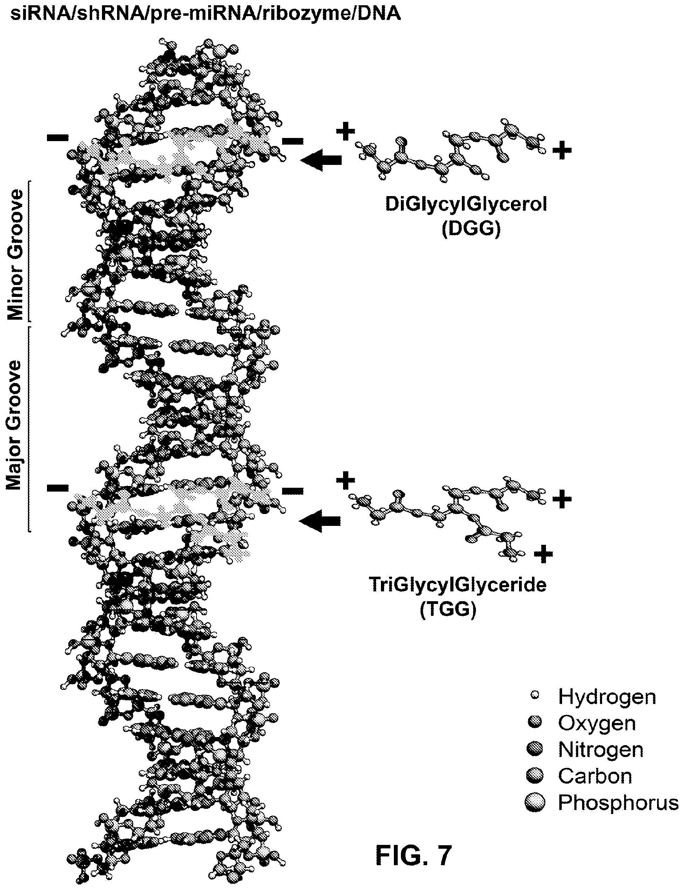

In principle, the present invention teaches a novel method of glycylation and its use for generating positively charged sugar alcohol and/or sugar compounds capable of interacting with negatively charged nucleic acid compositions via ionic binding and/or electrostatic affinity rather than covalent conjugation or hydrogen bonding (FIG. 7). Since such ionic/electrostatic affinity is formed between the glycyl groups of the modified sugars/sugar alcohols and the phosphodiester-linked backbones of RNAs/DNAs, the attached sugars/sugar alcohols can then repel water molecules away from the RNA/DNA backbones, so as to prevent hydrolysis and thus protect the intact structures of these nucleic acid compositions for better delivery of their drug effects into cells in vivo as well as in vitro.

In addition, the present invention discovered for the first time that chemical compounds containing sugar alcohols and/or sugars can protect the integrity of nucleic acid compositions, such as miRNA, shRNA, siRNA, ribozyme and DNA, from degradation. Hence, the present invention is not only a composition and its use for delivering nucleic acid-based drugs/vaccines into cells but also a material formula for preserving the structural integrity and functional efficacy of these nucleic acid-based drugs and/or vaccines in vivo as well as in vitro. Due to these novel features, the present invention is very useful for developing and/or improving a variety of nucleic acid-based cosmetic, pharmaceutical and therapeutic applications.

### BRIEF DESCRIPTION OF THE DRAWINGS:

Referring particularly to the drawings for the purpose of illustration only and not limitation, there is illustrated:
FIGs. 1A and 1B show the chemical reaction of glycylation and the resulting glycylated sugar alcohols thereof. For example, when glycerol (glycerin) is used as a model of sugar alcohol, two major reactive products for nucleic acid delivery can be identified: one is 1,3-diglycylglycerol (1A; DGG) and the other is 1,2,3-triglycylglyceride (1B; TGG). DGG is a partially glycylated product (1A), while TGG is a completely (or fully) glycylated product (1B).
FIGs. 2A-2D show the results of high performance liquid chromatography (HPLC) analyses before and after degradation of miR-302 precursors (*i.e.* pri- and pre-miR-302). FIG. 2A shows the comparison of HPLC patterns between fresh and degraded miR-302 precursors. The result of fresh sample (2A top) consists of total four peaks as follows: a small peak at 4.25 min (single nucleotide), a major peak at 9.50 min (1-hairpin pre-miR-302s), another small peak at 10.4 min (2-hairpin pre-miR-302s), and another major peak at 12.5 min (4-hairpin cluster pri-miRNA), whereas degraded sample (stored in normal saline for 3 days) displays a very smeared pattern (2A bottom). FIG. 2B shows the sequence of the miR-302 familial cluster gene from where the miR-302 precursors are transcribed and processed. FIG. 2C shows the individual sequences of 1-hairpin pre-miR-302 isoforms, including pre-miR-302a (SEQ.ID.NO.2), pre-miR-302b (SEQ.ID.NO.3), pre-miR-302c (SEQ.ID.NO.4), and pre-miR-302d (SEQ.ID.NO.5). FIG. 2D shows the comparison of HPLC patterns between fresh and sugar alcohol-protected miR-302 precursors, showing no degradation after 3-day storage in normal saline.
FIGs. 3A and 3B show the results of microRNA (miRNA) microarray analyses using small RNAs extracted from either non-treated blank keratinocytes or sugar alcohol (DGG/TGG)-mediated miR-302-transfected keratinocytes. FIG. 3A shows no miR-302 expression in blank keratinocytes, whereas FIG. 3B demonstrates that abundant miR-302 molecules were detected after sugar alcohol-mediated miR-302-transfection, indicating the success of sugar alcohol-based small RNA delivery into human cells.
FIGs. 4A-4C show the therapeutic effects of sugar alcohol-mediated anti-cancer drug (*i.e*. miR-302) delivery. Three different final concentrations of isolated miR-302 precursors (pri-/pre-miR-302s) at 0 (control), 200, and 400 µg/ml, respectively, were used to treat human liver cancer HepG2 cells. Also, in order to demonstrate the delivery efficiency of sugar alcohols after modification, we encapsulated these pri-/pre-miR-302s with sugar alcohols modified by four different levels of glycylation, including glycerin glycylated with 0 (control), 75, 750 and 1500 mg of glycine (Gly), respectively, which consequently result in a final MGG/DGG/TGG concentration of 0, ∼0.1, ∼0.5 and ∼1.0 M, respectively. As a result, FIG. 4A shows a significant dose-dependent increase of the miR-302-induced tumor suppression effects on human cancer cell growth following the increase of glycylated levels of MGG/DGG/TGG delivery (= Formulation 5). Meanwhile, FIG. 4B shows the treatments of MGG/DGG/TGG alone without miR-302 did not result in any effect or cytotoxicity. To further evaluate glycylated sugar/sugar alcohol-delivered miR-302 effects in vivo, FIG. 4C shows the therapeutic results of MGG/DGG/TGG-encapsulated pri-/pre-miR-302 treatments, resulting in an average >71% reduction in the sizes of human Huh-7 cancer xenografts grown on NOD SCID nude mice (n = 6).
FIG. 5 shows the in-vivo distribution of Cyanine 5.5 (Cy5.5)-labeled miR-302 mimics (siR-302) in mouse, wherein siR-302 consists of synthetic siRNA duplexes formed by the hybrids of 5'-Cy5.5-UAAGUGCUUC CAUGUUUUAG UGU-3' (SEQ.ID.NO.6) and 5'-Cy5.5-ACACUAAAAC AUGGAAGCAC UUA-3' (SEQ.ID.NO.7) in order to mimic the stem arm portion of pre-miR-302a/c.
FIG. 6 shows the survival rates of mice subjected to tail vein injection of either glycylated sugar alcohols only (200 µl of 1.0 M DGG/TGG; n = 4) or glycylated sugar alcohol-encapsulated siR-302 (200 µg of siR-302 in 200 µl of 1.0 M DGG/TGG; n = 4 after two sacrifices for toxicity examination and in-vivo siR-302 distribution analyses (6-2)).
FIG. 7 illustrates the interaction of electrostatic affinity between siRNA/shRNA/ miRNA/microRNA precursor/ribozyme/DNA and glycylated sugars/sugar alcohols (for example, DGG and TGG). The interaction area is labeled by green shadows to indicate the binding of DGG/TGG to the phosphate groups of RNA/DNA backbones. The phosphate groups of RNA/DNA are resided in the minor grooves of a nucleic acid strand. After multiple gylcylated sugars/sugar alcohols attach to the RNA/DNA strands, it forms a sugar/sugar alcohol coating on the encapsulated RNA/DNA, so as to protect the RNA/DNA structures and facilitate their delivery in vitro and in vivo.
FIG. 8 shows the separation of DGG/TGG from MGG using HPLC purification.

### EXAMPLES:

In the experimental disclosure which follows, the following abbreviations apply: M (molar); mM (millimolar); µm (micromolar); mol (moles); pmol (picomoles); gm (grams); mg (milligrams); µg (micrograms); ng (nanograms); L (liters); ml (milliliters); µl (microliters); °C (degrees Centigrade); RNA (ribonucleic acid); DNA (deoxyribonucleic acid); dNTP (deoxyribonucleotide triphosphate); PBS (phosphate buffered saline); NaCl (sodium chloride); HEPES (N-2-hydroxyethylpiperazine-N-2-ethanesulfonic acid); HBS (HEPES buffered saline); SDS (sodium dodecylsulfate); Tris-HCl (tris-hydroxymethylaminomethane-hydrochloride); ATCC (American Type Culture Collection, Rockville, MD); hESC (human embryonic stem cells); and iPSC (induced pluripotent stem cells).

### 1. MicroRNA (miRNA) Production and Isolation and siRNA Synthesis

Dicer-negative cells were acquired from Zymo Research (Irvine, CA), transduced with a pre-made miR-302 expression lentiviral vector *pLenti-EF1alpha-RGFP-miR302* (Mello Biotech, Santa Fe Springs, CA), and maintained according to manufacturers' suggestions. MicroRNAs were isolated with a *mir*Vana™ miRNA isolation kit (Ambion, Austin, TX), following the manufacturer's protocol. The isolated RNAs (pri-/pre-miR-302) were dissolved in autoclaved 1x TE buffer and stored at -80°C till use. For stability tests with HPLC, a desired amount of the isolated RNAs was re-collected with an Amicon Ultra-0.5mL 30K filter column (Millipore, Billerica, MA) and re-dissolved in autoclaved normal saline. For siR-302 preparation, synthetic miR-302 mimics were purchased from Sigma-Genosys (St. Louis, MO), containing two cyanine 5.5 (Cy5.5)-labeled RNA sequences: 5'-Cy5.5-UAAGUGCUUC CAUGUUUUAG UGU-3' (SEQ.ID.NO.6) and 5'-Cy5.5-ACACUAAAAC AUGGAAGCAC UUA-3' (SEQ.ID.NO.7). In experiments, siR-302 was formed by the hybrids of SEQ.ID.NO.6 and SEQ.ID.NO.7.

### 2. Glycylation of Sugar Alcohols and Formulation of miRNA/shRNA/siRNA

Although the natural way of sugar/sugar alcohol glycylation is unclear, we have developed a chemical procedure to artificially make glycylated sugar alcohols and sugars. First, a pre-made base solution was prepared, containing 1.0 M glycerol, 0.3 M fructose, and 0.9% (w/v) NaCl at about pH 2.5∼pH 8.0, depending on the source of sugar alcohol(s) used. We have noted that any sugar alcohol contaminated with alkaline materials might not work for glycylation. For activating glycylation, about a final concentration of 0.05-3.0 M glycine was added, depending on the desired concentration of final glycylated sugar alcohols/sugars, into the pre-made base solution and mixed well. Then, the glycine-added solution was kept strictly under a neutral to slightly acidic condition and incubated at a temperature over 45°C, preferably 75°C∼175°C, for >30 min, preferably about 12∼24 hours. Optionally, after that, 0.1 M mannitol was added to absorb any excessive glycine and the solution was further incubated over 45°C for another 12∼24 hours. When the final solution pH reached about 6.0 to 7.0, the glycylation of sugar alcohols/sugars was completed and this final solution was ready to be used for formulating and delivering negatively charged materials. The formulation is simply done by mixing the glycylated sugars/sugar alcohols with desired nucleic acid compositions. After that, the electrostatic/charge affinity between the glycylated sugar alcohols/sugars and nucleic acid compositions will form encapsulated delivery complexes (FIG. 7), which can then be absorbed by cells through active endocytosis. For demonstration, a schematic glycylation reaction using glycerol as an example was shown in FIGs. 1A and 1B.

### 3. Human Cell Culture and Transfection

Human keratinocytes was purchased from Invitrogen and cultivated in EpiLife serum-free cell culture medium supplemented with human keratinocyte growth supplements (HKGS, Invitrogen, Carlsbad, CA) in the absence of antibiotics at 37°C under 5% CO₂. Cells were passaged at about 50%-70% confluence by exposing the cells to trypsin/EDTA for about 1 min and then rinsing two times in HBSS containing trypsin inhibitor. The detached cells were replated at 1:5 dilution in fresh EpiLife medium with HKGS supplements. On the other hand, human liver cancer cell line HepG2 was obtained from ATCC and maintained according to manufacturer's suggestions. For miRNA transfection, pri-/pre-miR-302 prepared from Example 1 was dissolved in 0.1∼1.0 M of MGG/DGG/TGG solution at a desired concentration up to 5-10 mg/mL and then directly applied to cell culture medium based on the miRNA amount needed. For example, to deliver 200 µg pri-/pre-miR-302, we would need to add 40 µl of the MGG/DGG/TGG-dissolved pri-/pre-miR-302 (at 5 mg/ml) into the cell culture medium and then mix well with the cells. Since MGG/DGG/TGG is extremely safe and non-toxic, the tested cells could be cultivated in 0.1 M MGG/DGG/TGG with all necessary supplements and still not showing any adverse effect up to 48 hours. As a result, for cells growing in a 2-ml culture dish, the maximal transfection amount of miRNA/shRNA/siRNA with 0.1 M MGG/DGG/TGG is estimated to be about 10 mg, indicating a highly efficient delivery level that none of the previously reported liposomal and sugar-based delivery agents can achieve.

### 4. High Performance Liquid Chromatography (HPLC) Analysis

A reverse-phase HPLC method was developed for analyzing the purity and structural integrity of miR-302 and its precursors (*i.e.* pri-/pre-miR-302s). HPLC programs were run by an Ultimate 3000 HPLC machine (Thermo Scientific) with a DNA Pac PA-100 column (BioLC Semi-Prep 9x250 mm) at a flow rate of 3.6 ml/min. Starting buffer was 50 mM Tris-HCl (pH7.6) and mobile buffer was 50 mM Tris-HCl (pH7.6) with 500 mM sodium perchlorate. Signals of RNAs and DNAs were measured with an UV detector at 260 nm. The results were shown in FIGs. 2A and 2D as well as FIG. 8.

### 5. MicroRNA (miRNA) Microarray Analysis

At about 70% confluency, small RNAs from each cell culture were isolated, respectively, using the *mir*Vana™ miRNA isolation kit (Ambion). The purity and quantity of the isolated RNAs were assessed, using 1% formaldehyde-agarose gel electrophoresis and spectrophotometer measurement (Bio-Rad), and then immediately frozen in dry ice and submitted to LC Sciences (San Diego, CA) for miRNA microarray analyses. Each microarray chip was hybridized with a single sample labeled with either Cy3 or Cy5 or a pair of samples labeled with Cy3 and Cy5, respectively. Background subtraction and normalization were performed as manufacturer's suggestions. For a dual sample assay, a *p-*value calculation was performed and a list of differentially expressed transcripts more than 3-fold (yellow-red signals) was produced. The final microarray results were shown in FIGs. 3A and 3B, which compared the RNAs extracted from non-treated blank keratinocytes (3A) to those extracted from DGG/TGG-mediated miR-302-transfected keratinocytes (3B).

### 6. In Vivo Bio-Imaging of Sugar Alcohol-Delivered siRNA Distribution

To test the in-vivo deliver efficiency and toxicity of glycylated sugar alcohol-encapsulated nucleic acid compositions, we injected 200 µg of synthetic siR-302 (from Example 1) dissolved in 200 µl of 1.0 M DGG/TGG solution into each of C57BL/6J strain mice via tail vein injection. Approximately 24 hours post-injection, we sacrificed two mice for observing the DGG/TGG-delivered siR-302 distribution in vivo. Since these siR-302 molecules were labeled with infra-red fluorescent dye Cy5.5, we could directly observe their in-vivo distribution using a bio-imaging system and/or their fluorescent signals in mouse tissue sections under a fluorescent microscope. The results were shown in FIG. 5.

### 7. Statistic Analysis

Any change over 75% of signal intensity was considered as a positive result, which in turn was analyzed and presented as mean ± SE. Statistical analysis of data was performed by one-way ANOVA. When main effects were significant, the Dunnett's post-hoc test was used to identify the groups that differed significantly from the controls. For pairwise comparison between two treatment groups, the two-tailed student *t* test was used. For experiments involving more than two treatment groups, ANOVA was performed followed by a post-hoc multiple range test. Probability values of *p* <0.05 was considered significant. All *p* values were determined from two-tailed tests.

### REFERENCES:

1. Immordino ML, Dosio F, Cattel L. (2006) Int J Nanomedicine. Stealth liposomes: review of the basic science, rationale, and clinical applications, existing and potential. Int J Nanomedicine 1, 297-315.
2. WIPO Patent No. WO2011143237 to Meyering.
3. Pereira GR, Collett JH, Garcia SB, Thomazini JA, Bentley MVLB. (2002) Glycerol monooleate/solvents systems for progesterone transdermal delivery: in vitro permeation and microscopic studies. Brazilian Journal of Pharmaceutical Sciences 38, 55-62.
4. Zhen G, Hinton TM, Muir BW, Shi S, Tizard M, McLean KM, Hartley PG, Gunatillake P. (2012) Glycerol monooleate-based nanocarriers for siRNA delivery in vitro. Mol Pharm. 9, 2450-2457.
5. Gao H, Elsabahy M, Giger EV, Li D, Prud'homme RE, Leroux JC. (2010) Aminated linear and star-shape poly(glycerol methacrylate)s: synthesis and self-assembling properties. Biomacromolecules. 11, 889-895.
6. Gao H, Lu X, Ma Y, Yang Y, Li J, Wu G, Wang Y, Fan Y, Ma J. (2011) Amino poly(glycerol methacrylate)s for oligonucleic acid delivery with enhanced transfection efficiency and low cytotoxicity. Soft Matter 7, 9239-9247.
7. European Patent Application No. EP92116370.5 to Nair.
8. WIPO Patent No. WO2009029046 to Kim.
9. U.S. Patent Application No. 5,618,933 to Dordick.
10. Banerjee G, Nandi G, Mahato SB, Pakrashi A, Basu MK. (1996) Drug delivery system: targeting of pentamidines to specific sites using sugar grafted liposomes. Journal of Antimicrobial Chemotherapy 38, 145-150.
11. WIPO Patent No. WO 2002032398 to Kohane.
12. Davis BG and Robinson MK. (2002) Drug delivery systems based on sugar-macromolecule conjugates. Current Opinion in Drug Discovery & Development 5, 279-288.
13. Blanchfield J and Toth I. (2004) Lipid, sugar and liposaccharide based delivery systems 2. Current Medicinal Chemistry 11, 2375-2382.
14. Morris GA, Kok MS, Harding SE, Adams GG. (2010) Polysaccharide drug delivery systems based on pectin and chitosan. Biotechnology and Genetic Engineering Reviews 27, 257-284.
15. Cuña M, Alonso-Sandel M, Remuñán-López C, Pivel JP, Alonso-Lebrero JL, Alonso MJ. (2006) Development of phosphorylated glucomannan-coated chitosan nanoparticles as nanocarriers for protein delivery. J Nanosci Nanotechnol. 6, 2887-2895.
16. Graf A, Ablinger E, Peters S, Zimmer A, Hook S, Rades T. (2008) Microemulsions containing lecithin and sugar-based surfactants: nanoparticle templates for delivery of proteins and peptides. Int J Pharm. 350, 351-360.
17. Davis ME, Zuckerman JE, Choi CH, Seligson D, Tolcher A, Alabi CA, Yen Y, Heidel JD, Ribas A. (2010) Evidence of RNAi in humans from systemically administered siRNA via targeted nanoparticles. Nature 464, 1067-1070.
18. Bhatia S, Mohr A, Mathur D, Parmar VS, Haag R, Prasad AK. (2011) Biocatalytic route to sugar-PEG-based polymers for drug delivery applications. Biomacromolecules 12, 3487-3498.
19. Ellis GA, Palte MJ, Taines RT. (2012) Boronate-Mediated Biologic Delivery. Journal of American Chemical Society 134, 3631-3634.
20. Lin SL, Jiang A, Chang D, and Ying SY. (2008) Loss of mir-146a function in hormone-refractory prostate cancer. RNA 14, 417-424.
21. Lin SL, Chang D, Chang-Lin S, Lin CH, Wu DTS, Chen DT, and Ying SY. (2008) Mir-302 reprograms human skin cancer cells into a pluripotent ES-cell-like state. RNA 14,2115-2124.
22. Lin SL, Chang D, Ying SY, Leu D, and Wu DTS. (2010) MicroRNA miR-302 inhibits the tumorigenecity of human pluripotent stem cells by coordinate suppression of CDK2 and CDK4/6 cell cycle pathways. Cancer Res. 70, 9473-9482.
23. Chen SKJ and Lin SL. (2013) Recent patents on microRNA-induced pluripotent stem cell generation. Recent Patents on Regenerative Medicine 3, 5-16.

## Claims

1. A composition for formulating nucleic acid compositions with sugars and sugar alcohols as stable complexes for both in-vitro and in-vivo delivery into mammalian cells, comprising:
(a) at least a nucleic acid composition with at least a negative charge;
(b) at least one sugar or sugar alcohol composition modified by glycylation;
wherein the sugar alcohol has a formula of H(HCHO)ₙ₊₁H, and the sugar has a formula of H(HCHO)ₙHCO, (a) and (b) are mixed together under a condition to form at least a delivery complex.

2. The composition of claim 1, wherein said nucleic acid composition is RNA or DNA.

3. The composition of claim 2, wherein said nucleic acid composition is selected from the group consisting of miRNA, microRNA precursors, small hairpin RNAs (shRNA), short interfering RNAs (siRNA), ribozymes, antisense RNAs/DNAs, RNA-DNA hybrids, DNA vectors/vaccines, and a combination thereof.

4. The composition of claim 1, wherein said sugar or sugar alcohol composition is selected from the group consisting of glucose, fructose, galactose, sucrose, lactose, alditol, arabitol, erythritol, fucitol, galactitol, glycerol (glycerin), iditol, inositol, isomalt, lactitol, maltitol, mannitol, polyglycitol, sorbitol, threitol, volemitol, xylitol, and a combination thereof.

5. The composition of claim 1, wherein said at least one modified sugar or sugar alcohol composition by glycylation contains a mixture of diglycylglycerol and triglycylglyceride.

6. The composition of claim 1, wherein said modified sugar or sugar alcohol composition by glycylation is positively charged.

7. The composition of claim 1, wherein the concentration of said modified sugar or sugar alcohol composition for formulating nucleic acid compositions ranges from 0.1 µM to 10 M.

8. The composition of claim 1, wherein a maximal solubility of said nucleic acid composition in the modified sugar or sugar alcohol composition in a solution is up to 15 mg/mL.

9. The composition of claim 1, wherein said modified sugar or sugar alcohol composition protects said nucleic acid compositions from degradation.

10. The composition of claim 1, wherein said modified sugar or sugar alcohol composition enhances the delivery efficiency of said nucleic acid compositions into mammalian cells in vitro as well as in vivo.

11. The composition of claim 1, wherein said condition is a pH value below 8.0.

12. The composition of claim 11, wherein said condition is a pH value between pH 2.5 and pH 7.0.

13. The composition of claim 1, wherein said delivery complex is formed by the ionic or electrostatic affinity occurring between the modified sugar or sugar alcohol composition and the nucleic acid composition.

14. The composition of claim 1, wherein said glycylation is a chemical reaction in that at least one hydroxyl group of the sugar or the sugar alcohol is replaced by at least one glycyl group from a glycine or a glycylated amino acid.

15. The composition of any one of the preceding claims, wherein said delivery complex is for the use in cosmetic, pharmaceutical and therapeutic applications.

16. A method for delivering nucleic acids into cells in vitro, comprising:
mixing a mixture of at least a sugar or sugar alcohol with hydroxyl group and at least an amino acid with a structure of glycyl group, and
mixing said mixture and at least a nucleic acid composition to form a formulation composition, wherein said formulation composition comprises at least one glycylated sugar or glycylated sugar alcohol which interacts with said nucleic acid composition via ionic or electrostatic affinity and forms a delivery complex at a pH value below 8.0; and
applying said delivery complex to the cells.

## Patentansprüche

1. Zusammensetzung zum Formulieren von Nucleinsäurezusammensetzungen mit Zucker und Zuckeralkoholen als stabile Komplexe für sowohl In-vitro- als auch In-vivo-Zuführung zu Säugetierzellen, die Folgendes umfasst:
(a) wenigstens eine Nucleinsäurezusammensetzung mit wenigstens einer negativen Ladung;
(b) wenigstens eine durch Glycylierung modifizierte Zucker- oder Zuckeral koholzusammensetzung;
wobei der Zuckeralkohol die Formel H(HCHO)ₙ₊₁H hat und der Zucker die Formel H(HCHO)ₙHCO hat, (a) und (b) unter einer Bedingung miteinander gemischt werden, um wenigstens einen Zuführungskomplex zu bilden.

2. Zusammensetzung nach Anspruch 1, wobei die genannte Nucleinsäurezusammensetzung RNA oder DNA ist.

3. Zusammensetzung nach Anspruch 2, wobei die genannte Nucleinsäurezusammensetzung ausgewählt ist aus der Gruppe bestehend aus miRNA, microRNA-Vorläufern, Small hairpin RNAs (shRNA), Short interfering RNAs (siRNA), Ribozymen, Antisense-RNAs/DNAs, RNA-DNA-Hybriden, DNA-Vektoren/Impfstoffen und einer Kombination davon.

4. Zusammensetzung nach Anspruch 1, wobei die genannte Zucker- oder Zuckeralkoholzusammensetzung ausgewählt ist aus der Gruppe bestehend aus Glucose, Fructose, Galactose, Saccharose, Lactose, Alditol, Arabitol, Erythritol, Fucitol, Galactitol, Glycerol (Glycerin), Iditol, Inositol, Isomalt, Lactitol, Maltitol, Mannitol, Polyglycitol, Sorbitol, Threitol, Volemitol, Xylitol und einer Kombination davon.

5. Zusammensetzung nach Anspruch 1, wobei die genannte wenigstens eine durch Glycylierung modifizierte Zucker- oder Zuckeralkoholzusammensetzung ein Gemisch aus Diglycylglycerol und Triglycylglycerid enthält.

6. Zusammensetzung nach Anspruch 1, wobei die genannte durch Glycylierung modifizierte Zucker- oder Zuckeralkoholzusammensetzung positiv geladen ist.

7. Zusammensetzung nach Anspruch 1, wobei die Konzentration der genannten modifizierten Zucker- oder Zuckeralkoholzusammensetzung zur Formulierung von Nucleinsäurezusammensetzungen im Bereich von 0,1 µM bis 10 M liegt.

8. Zusammensetzung nach Anspruch 1, wobei eine maximale Löslichkeit der genannten Nucleinsäurezusammensetzung in der modifizierten Zucker- oder Zuckeralkoholzusammensetzung in einer Lösung bis zu 15 mg/ml beträgt.

9. Zusammensetzung nach Anspruch 1, wobei die genannte modifizierte Zucker- oder Zuckeralkoholzusammensetzung die genannten Nucleinsäurezusammensetzungen vor Abbau schützt.

10. Zusammensetzung nach Anspruch 1, wobei die genannte modifizierte Zucker- oder Zuckeralkoholzusammensetzung die Zuführungsleistung der genannten Nucleinsäurezusammensetzungen zu Säugerzellen in vitro sowie in vivo erhöht.

11. Zusammensetzung nach Anspruch 1, wobei die genannte Bedingung ein pH-Wert unter 8,0 ist.

12. Zusammensetzung nach Anspruch 11, wobei die genannte Bedingung ein pH-Wert zwischen 2,5 und 7,0 ist.

13. Zusammensetzung nach Anspruch 1, wobei der genannte Zuführungskomplex durch die ionische oder elektrostatische Affinität gebildet wird, die zwischen der modifizierten Zucker- oder Zuckeralkoholzusammensetzung und der Nucleinsäurezusammensetzung auftritt.

14. Zusammensetzung nach Anspruch 1, wobei die genannte Glycylierung eine chemische Reaktion ist, bei der wenigstens eine Hydroxylgruppe des Zuckers oder des Zuckeralkohols durch wenigstens eine Glycylgruppe von einem Glycin oder einer glycylierten Aminosäure ersetzt wird.

15. Zusammensetzung nach einem der vorherigen Ansprüche, wobei der genannte Zuführungskomplex zur Verwendung in kosmetischen, pharmazeutischen und therapeutischen Anwendungen vorgesehen ist.

16. Verfahren zum Zuführen von Nucleinsäuren zu Zellen in vitro, das Folgendes beinhaltet:
Mischen eines Gemischs aus wenigstens einem Zucker oder Zuckeralkohol mit einer Hydroxylgruppe und wenigstens einer Aminosäure mit einer Struktur einer Glycylgruppe, und
Mischen des genannten Gemischs und wenigstens einer Nucleinsäurezusammensetzung, um eine Formulierungszusammensetzung zu bilden, wobei die genannte Formulierungszusammensetzung wenigstens einen glycylierten Zucker oder glycylierten Zuckeralkohol umfasst, der mit der genannten Nucleinsäurezusammensetzung über ionische oder elektrostatische Affinität interagiert und einen Zuführungskomplex bei einem pH-Wert unter 8,0 bildet; und
Anwenden des genannten Zuführungskomplexes an den Zellen.

## Revendications

1. Une composition pour la formulation de compositions d'acide nucléique avec des sucres ou des alcools de sucres en complexes stables pour une administration in-vitro ainsi qu'in-vivo dans des cellules de mammifères, comprenant :
(a) au moins une composition d'acide nucléique avec au moins une charge négative ;
(b) au moins une composition de sucre ou d'alcool de sucre modifiée par glycylation ;
dans laquelle l'alcool de sucre a une formule de H(HCHO)ₙ₊₁H, et le sucre a une formule de H(HCHO)ₙHCO, (a) et (b) sont mélangés ensemble selon une condition pour former au moins un complexe d'administration.

2. La composition de la revendication 1, dans laquelle ladite composition d'acide nucléique est un ARN ou un ADN.

3. La composition de la revendication 2, dans laquelle ladite composition d'acides nucléiques est sélectionnée dans le groupe consistant en miARN, précurseurs de microARN, petits ARN en épingle à cheveux (shRNA), petits ARN interférents (siRNA), ribozymes, ARN/ADN antisens, hybrides ARN-ADN, vecteurs/vaccins ADN, et une combinaison de ceux-ci.

4. La composition de la revendication 1, dans laquelle ladite composition de sucre ou d'alcool de sucre est sélectionnée dans le groupe consistant en glucose, fructose, galactose, sucrose, lactose, alditol, arabitol, érythritol, fucitol, galactitol, glycérol (glycérine), iditol, inositol, isomalt, lactitol, maltitol, mannitol, polyglycitol, sorbitol, thréitol, volémitol, xylitol, et une combinaison de ceux-ci.

5. La composition de la revendication 1, dans laquelle ladite au moins une composition de sucre ou d'alcool de sucre modifiée par glycylation contient un mélange de diglycylglycérol et triglycylglycéride.

6. La composition de la revendication 1, dans laquelle ladite composition de sucre ou d'alcool de sucre modifiée par glycylation est chargée positivement.

7. La composition de la revendication 1, dans laquelle la concentration de ladite composition de sucre ou d'alcool de sucre modifiée pour formuler des compositions d'acide nucléique est située dans une plage allant de 0,1 µM à 10 M.

8. La composition de la revendication 1, dans laquelle une solubilité maximum de ladite composition d'acide nucléique dans la composition de sucre ou d'alcool de sucre modifiée dans une solution va jusqu'à 15 mg/ml.

9. La composition de la revendication 1, dans laquelle ladite composition de sucre ou d'alcool de sucre modifiée protège lesdites compositions d'acide nucléique de la dégradation.

10. La composition de la revendication 1, dans laquelle ladite composition de sucre ou d'alcool de sucre améliore l'efficacité d'administration desdites compositions d'acide nucléique dans des cellules de mammifères in vitro ainsi qu'in vivo.

11. La composition de la revendication 1, dans laquelle ladite condition est une valeur de pH inférieure à 8,0.

12. La composition de la revendication 11, dans laquelle ladite condition est une valeur de pH située entre pH 2,5 et pH 7,0.

13. La composition de la revendication 1, dans laquelle ledit complexe d'administration est formé par l'affinité ionique ou électrostatique se produisant entre la composition de sucre ou d'alcool de sucre modifiée ou la composition d'acide nucléique.

14. La composition de la revendication 1, dans laquelle ladite glycylation est une réaction chimique dans laquelle au moins un groupe hydroxyle du sucre ou de l'alcool de sucre est remplacé par au moins un groupe glycyle venant d'une glycine ou d'un acide aminé glycylé.

15. La composition de n'importe laquelle des revendications précédentes, dans laquelle ledit complexe d'administration est destiné à l'usage dans des applications cosmétiques, pharmaceutiques et thérapeutiques.

16. Un procédé d'administration d'acides nucléiques dans des cellules in vitro, comprenant les étapes consistant à :
mélanger un mélange d'au moins un sucre ou alcool de sucre avec un groupe hydroxyle et au moins un acide aminé ayant une structure de groupe glycyle, et
mélanger ledit mélange et au moins une composition d'acide nucléique pour former une composition de formulation, ladite composition de formulation comprenant au moins un sucre glycylé ou alcool de sucre glycylé qui interagit avec ladite composition d'acide nucléique via une affinité ionique ou électrostatique et forme un complexe d'administration ayant une valeur de pH inférieure à 8,0 ; et
appliquer ledit complexe d'administration aux cellules.
